(19) Europäisches Patentamt
European Patent Office
Office européen des brevets

(11) **EP 2 835 143 B1**

(12) **EUROPÄISCHE PATENTSCHRIFT**

(45) Veröffentlichungstag und Bekanntmachung des Hinweises auf die Patenterteilung:
**19.12.2018 Patentblatt 2018/51**

(51) Int Cl.:
***A61M 1/14*** *(2006.01)*      ***G16H 50/50*** *(2018.01)*

(21) Anmeldenummer: **14179679.7**

(22) Anmeldetag: **04.08.2014**

(54) **Vorrichtung zur Vorhersage intradialytischer Parameter**

Device for predicting intradialytic parameters

Dispositif de prédiction de paramètres intradialytiques

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.08.2013 DE 102013108543**

(43) Veröffentlichungstag der Anmeldung:
**11.02.2015 Patentblatt 2015/07**

(73) Patentinhaber: **B. Braun Avitum AG**
**34212 Melsungen (DE)**

(72) Erfinder:
• **Attalah, Richard**
**34125 Kassel (DE)**
• **Henze, Janosch**
**34121 Kassel (DE)**
• **Strohhöfer, Christof, Dr.**
**34123 Kassel (DE)**

(74) Vertreter: **Winter, Brandl, Fürniss, Hübner,**
**Röss, Kaiser, Polte - Partnerschaft mbB**
**Patent- und Rechtsanwaltskanzlei**
**Alois-Steinecker-Strasse 22**
**85354 Freising (DE)**

(56) Entgegenhaltungen:
WO-A1-2010/028860      US-A1- 2007 175 827
US-A1- 2012 273 354      US-A1- 2012 277 551

• **FERNANDEZ E A ET AL: "Dialysate-side urea kinetics. Neural network predicts dialysis dose during dialysis", MEDICAL & BIOLOGICAL ENGINEERING & COMPUTING, SPRINGER, BERLIN, DE, Bd. 41, Nr. 4, 1. Juli 2003 (2003-07-01), Seiten 392-396, XP019834524, ISSN: 1741-0444**
• **HONGWEI LIU: "On the Levenberg-Marquardt training method for feed-forward neural networks", NATURAL COMPUTATION (ICNC), 2010 SIXTH INTERNATIONAL CONFERENCE ON, IEEE, PISCATAWAY, NJ, USA, 10. August 2010 (2010-08-10), Seiten 456-460, XP031761478, ISBN: 978-1-4244-5958-2**
• **SAFAVIEH E ET AL: "Forecasting the Unknown Dynamics in NN3 Database Using a Nonlinear Autoregressive Recurrent Neural Network", NEURAL NETWORKS, 2007. IJCNN 2007. INTERNATIONAL JOINT CONFERENCE ON, IEEE, PISCATAWAY, NJ, USA, 1. August 2007 (2007-08-01), Seiten 2105-2109, XP031154919, ISBN: 978-1-4244-1379-9**
• **DERRICK MIRIKITANI ET AL: "Recursive Bayesian Levenberg-Marquardt Training of Recurrent Neural Networks", NEURAL NETWORKS, 2007. IJCNN 2007. INTERNATIONAL JOINT CONFERENCE ON, IEEE, PISCATAWAY, NJ, USA, 1. August 2007 (2007-08-01), Seiten 282-287, XP031154605, DOI: 10.1109/IJCNN.2007.4371045 ISBN: 978-1-4244-1379-9**

**Beschreibung**

Die Erfindung betrifft eine Vorrichtung zur Vorhersage intradialytischer Parameter, nämlich eines Blutdrucks. Bisher wurden Behandlungsprobleme wie z.B. intradialytische Hypotonien durch retrospektive Regelung reduziert oder behoben. Ein Eingriff, wie z.B. die Reduzierung der Ultrafiltrationsrate (UF-Rate), wurde also nur beim Auftreten einer hypotensiven Episode durchgeführt.

Symptomatische, intradialytische Hypotonien gehören zu den am meisten auftretenden Komplikationen während einer Dialysebehandlung. Eine der Hauptursachen dieser Hypotonien ist das Ungleichgewicht zwischen der UF-Rate und dem Refill-Prozess, d.h. das Nachströmen von Wasser aus dem intrazellulären Raum und dem Interstitium in den intravasalen Raum. Zur Verringerung und Vermeidung solcher Hypotonien wurden verschiedene Techniken entwickelt, die auf der Basis von Biofeedbacksystemen beruhen. Mögliche Regelungsparameter zur Stabilisierung des hämodynamischen Kreislaufs des Patienten sind z.B. der Blutdruck BD, und das relative Blutvolumen RBV. Als Stellparameter kommen die Dialysierflüssigkeitstemperatur DT, die Ultrafiltrationsrate UFR bzw. UF-Rate und die Dialysierflüssigkeitsleitfähigkeit LF, in Frage. Die erwähnten Regelungs- und Stellparameter treten in unterschiedlichen Kombinationen in Wechselwirkung zusammen.

Ein in der EP 0 956 872 A2, EP 1 226 838 A2 und EP 1 844 800 B1 beschriebenes Biofeedback-System regelt die direkte Ursache von Hypotonien, den Blutdruck.

In WO 2011/080185 und WO 2011/080190 ist eine Messeinrichtung zur Vorhersage eines Blutdruck-Abfalls beschrieben. Dabei wird mittels verschiedener, von der Maschine und / oder direkt am Patienten aufgenommener Signale eine Pulsamplitude berechnet, die zu einer Varianz führt. Diese Varianz liefert Erkenntnisse über das Auftreten einer Hypotension.

In der EP 2 061 532 B1 ist eine Therapieeinrichtung mit gedächtnisgestützter Regeleinrichtung beschrieben, wobei die Verläufe des Blutdrucks in vorangegangenen Therapien auf Ähnlichkeit mit dem aktuellen Blutdruck-Verlauf zum aktuellen Zeitpunkt untersucht werden. Aus den Kurven mit der höchsten Ähnlichkeit wird mindestens eine Kurve mit einem Blutdruck-Abfall selektiert, die die Führungsgröße der vorausschauenden Blutdruck-Regelung übernimmt. Dabei besteht allerdings die Möglichkeit, dass bei dem Test eine Kurve mit einer sehr geringen Ähnlichkeit ausgewählt wird, die als Leitkurve für die aktuelle Therapie gilt.

Die oben erwähnten Biofeedbacksysteme reagieren allerdings lediglich retrospektiv, d.h. erst nach dem Auftreten einer hypotensiven Episode. Ein Blutdruck-Abfall kann hierdurch nicht vorhergesagt werden.

Bei der Regelung des Blutvolumens wird dieses kontinuierlich gemessen und gezwungen, anhand von UF- und LF-Steuerung einem vordefinierten Blutvolumenverlauf zu folgen, um den das Blutvolumen nur in einem vordefinierten Intervall schwanken darf. Das wird anhand einer MIMO (Multi Input Multi Output) adaptiven Regelung realisiert.

Bei einem anderen Biofeedback-System werden die arterielle und venöse Temperatur durch in der Maschine eingebaute Temperatursensoren aufgenommen. Durch Änderung der Dialysierflüssigkeitstemperatur wird der Unterschied der aufgenommen arteriellen und venösen Temperatur auf den Sollwert geregelt. Infolgedessen findet eine Änderung der venösen bzw. der extrakorporalen Temperatur statt. Dadurch könnte man einen stabilen Patientenzustand erreichen. Die Regelung der Temperatur gibt aber keine direkte Information über den aktuellen Zustand des Patienten.

In der EP 0 956 872 A2 (EP 0956 872 B1) ist eine Regelung des Blutdrucks beschrieben, die auf der Überwachung des Blutdrucks des Patienten in gewissen Zeitabständen beruht. Je nach Blutdruck-Verlauf und nach aktuellem Blutdruck in Abhängigkeit von zwei vorgegebenen Grenzen wird die UF-Rate gestellt. Bei diesem Biofeedbacksystem wird auch retrospektiv reagiert. Die UF-Rate wird erst gestellt, nachdem Blutdruck-Abfälle detektiert wurden.

In EP 1 844 800 B1 ist eine zeitflexible Regelung des Blutdrucks beschrieben, bei der zu festgelegten Zeitpunkten Blutdruck-Werte aus patienteneigenen, vorherigen Blutdruck-Verläufen substituiert werden. Hierdurch kann die Anzahl von Blutdruck-Messungen innerhalb einer Therapie reduziert werden.

EP 0956 872 A2 (EP 0956 872 B1), EP 1 844 800 B1 und EP 2 061 532 B1 benötigen jeweils eine gewisse Anzahl an aktuellen Blutdruck-Messungen, damit der Vergleich mit vorangegangenen Therapien gestartet werden kann. Dies führt zu einem Nachteil, dass bis dahin ein Blutdruck-Abfall auftreten kann.

In der WO 2011/080185 und WO 2011/080190 ist eine Vorhersage eines Blutdruck-Abfalls beschrieben, auf den durch ein Warnsignal hingewiesen wird. Durch dieses Signal wird das Personal informiert, um Gegenmaßnahmen zu ergreifen. Ist die Vorhersage einer Hypotension nicht korrekt, wird das Personal unnötig informiert, was zu einer Zeitverschwendung und einer Belastung des Patienten bei Gegenmaßnahmen, wie z.B. Trendelenburg - Position, führt.

Die US 2007/0175827 A1 offenbart ein Patientenüberwachungssystem, welches physiologische Parameter eines Patienten misst und an ein Host-System überträgt. Offenbarungsgemäß kann eine Hypotonie vorhergesagt werden. Außerdem können patientenspezifische Grenzwerte für Hypotonien bestimmt werden.

Der Erfindung liegt die Aufgabe zugrunde, eine Vorrichtung bereit zu stellen, die die Behandlungsqualität bei der Behandlung erhöhen kann. Mit der Erfindung wird eine Vorrichtung gemäß dem Patentanspruch 1 geschaffen.

Ausführungsbeispiele der Erfindung betreffen die Vorhersage intradialytischer Parameter, wie z.B. BD. Die Vorhersage kann mit Hilfe von Lernalgorithmen, wie z.B. mit neuronalen Netzen, stattfinden. Diese Vorhersage ermöglicht dem Personal einen Blick über das Verhalten der Patientenparameter in die Zukunft, so dass frühzeitige Maßnahmen ergriffen werden können, oder ein rechtzeitiger automatischer Eingriff durch die Maschine erfolgen kann.

Gemäß einem Aspekt der Erfindung ist eine Vorrichtung zur Vorhersage intradialytischer Parameter wie z.B. eines Blutdrucks geschaffen, wobei mindestens ein Lernalgorithmus und / oder mindestens ein neuronales Netz vorgesehen ist. In einer Speichereinrichtung sind patientenindividuellen intradialytischen Parametern, Laborparametern und / oder Maschinenparametern speicherbar, die bei der Vorhersage von patienteneigenen Parameterverläufen während einer Dialysebehandlung einsetzbar sind.

Die patientenindividuellen intradialytischen Parameter können z.B. der Blutdruck und / oder das relative Blutvolumen, RBV, sein. Die Laborparameter sind z.B. Albumin und / oder Harnstoff. Die Maschinenparameter sind z.B. ein venöser Druck, PV, und / oder ein arterieller Druck, PA.

Vorteilhafterweise kann ein Trend über den Verlauf des gewünschten Parameters angezeigt werden, z.B. optisch oder akustisch, so dass das Behandlungspersonal unmittelbar informiert werden kann.

Mit einem Biofeedbacksystem, das die Vorhersage als Input verwendet, ist es möglich rasch automatisch Gegenmaßnahmen, z.B. in Form einer UF-Reduktion, einer LF-Änderung, einer Dialysierflüssigkeitstemperatur-Änderung oder einer Injektion einer isotonischen Lösung zu ergreifen.

Vorzugsweise kann mindestens eine Sensor- oder Speicheranordnung zur Erfassung oder Speicherung von Maschinenparametern wie etwa einem venösen Druck PV, einem arteriellen Druck PA, einem Transmembrandruck TMP, einer Leitfähigkeit LF der Dialysierflüssigkeit, einer Dialysierflüssigkeitstemperatur DT und / oder weiterer Parameter, und / oder eine Sensoranordnung oder Speicheranordnung zur Erfassung von Patientenparametern wie etwa einer Absorbanz von urämischen Toxinen, des Hämatokrit HCT, und / oder eine Sensoranordnung bzw. Speicheranordnung zur Erfassung oder Speicherung von Laborparametern wie etwa von Albumin, Harnstoff usw. vorgesehen sein.

Mit der vorgesehenen Trainingseinheit und Vorhersageeinheit lässt sich die Vorhersagegenauigkeit ermitteln, wobei die Trainingseinheit vorzugsweise dazu ausgelegt ist, zunächst eine Lernphase durchzuführen, wonach sie die entsprechend trainierten, d.h. entsprechend angepassten Werte eines Trainingsalgorithmus an die Vorhersageeinheit abgibt. Die Vorhersageeinheit kann dann durch den integrierten Trainingsalgorithmus eine Prädiktion aufgrund von Extrapolationen oder anderweitigen Berechnungen für die Vorhersage des zukünftigen Verlaufs von zu ermittelnden oder überwachenden Parametern durchführen. Die Vorhersageparameter können ausgewertet, mit Schwellwerten verglichen und / oder zur Anzeige gebracht werden, so dass eine optimierte rasche Auswertung und Information erreichbar ist.

Vorzugsweise können die Trainingseinheit, die Vorhersageeinheit und / oder eine Vorhersageparameter bildende Einrichtung jeweils als künstliches neuronales Netz ausgelegt sein. Alternativ oder zusätzlich können Support Vector Machines, Stützvektormaschinen, eingesetzt werden.

Optional kann ein Alarmgeber zur Ausgabe einer Warnung z.B. in akustischer und / oder optischer Form, und / oder einer Anzeige vorgesehen sein, auf der der aktuelle Verlauf der Vorhersageparameter einschließlich des zukünftig zu erwartenden, geschätzten Verlaufs darstellbar ist.

Bei einem oder mehreren Ausführungsbeispielen ist ein Regler vorgesehen, der auf der Basis der Vorhersageparameter sowie auf der Basis der aktuellen Parameter, beispielsweise des Blutdrucks oder des relativen Blutvolumens, Regeleingriffe vornehmen kann, so dass ein rascher präziser Korrektureingriff bei Bedarf erfolgen kann.

Während einer anfänglichen Phase kann die Struktur eines neuronalen Netzwerks mit einer Eingangsschicht mit mindestens zwei Eingängen, mindestens einer verborgenen Schicht mit mindestens drei Neuronen, und mindestens einer Ausgangsschicht mit mindestens einem Ausgang angepasst und trainiert werden und die Ausgänge an Eingangsdaten angepasst werden.

Hierbei können Gewichte des vordefinierten Netzwerks berechnet und aktualisiert werden.

Auch die Struktur des Netzwerks, z.B. die Anzahl der Neuronen oder von Hidden Layers, kann automatisch verändert werden, um ein optimiertes Lernen mit einem vorhandenen Datensatz zu gewährleisten, und den Lernalgorithmus anzupassen.

Als Trainingsalgorithmen kann ein Training der Netzwerke mit dem *Bayesian Regulation-Backpropagation*- und mit dem *Levenberg-Marquardt-Backpropagation*-Algorithmus vorgesehen sein. Für den Trainingsalgorithmus kann entweder der NAR- oder der NARX-Network verwendet werden.

Eine weitere Verbesserung der Vorhersage lässt sich durch Heranziehung mindestens eines oder mehrerer der folgenden Inputparameter neben dem Blutdruck erreichen, nämlich Ultrafiltrationsrate, UF-Rate; Ultrafiltrationsvolumen; arterieller und venöser Druck; Hämatokrit; relatives Blutvolumen; Sauerstoffsättigung; Hämoglobin; Ultrafiltrationsrate; Herzrate; Absorbanz urämischer Toxine; systolischer Blutdruck; Dialysierflüssigkeitsleitfähigkeit (Säure und Base Leitfähigkeit, PH-Wert), und / oder Dialysierflüssigkeitstemperatur, und / oder weiterer Lerntechniken erzielen.

Ein weiterer Aspekt der Offenbarung betrifft ein Verfahren zur Vorhersage intradialytischer Parameter wie z.B. eines Blutdrucks, wobei mindestens ein Lernalgorithmus und / oder mindestens ein neuronales Netz vorgesehen ist, patientenindividuelle intradialytische Parameter, Laborparameter und / oder Maschinenparametern gespeichert und bei der Vorhersage von patienteneigenen Parameterverläufen während einer Dialysebehandlung eingesetzt werden. Die patientenindividuellen intradialytischen Parameter können der Blutdruck und / oder das relative Blutvolumen, RBV, sein. Die Laborparameter sind z.B. Albumin und / oder Harnstoff und / oder die Maschinenparameter sind z.B. ein venöser Druck, PV, und / oder ein arterieller Druck, PA. Ein Trend über den Verlauf des gewünschten Parameters oder der Verlauf selbst kann angezeigt werden, z.B. optisch oder akustisch.

Durch die Speicherung von patientenindividuellen intradialytischen Parametern (z.B. BD, RBV,...), Laborparametern (z.B. Albumin, Harnstoff, ...) und Maschinenparametern (z.B. venöser Druck PV, arterieller Druck PA, ...) können patienteneigene Parameterverläufe während einer Dialysebehandlung vorhergesagt werden. Ein hier relevanter Parameter kann der Blutdruck sein. Diese Vorhersage gibt dem Anwender zukünftige Informationen über den Zustand des Patienten, so dass der Anwender (z.B. das Personal) frühzeitig mit einer Umstellung eines oder mehrerer Dialyse- oder Patientenparameters interagieren kann. Wird der Blutdruck-Verlauf des Patienten vorhergesagt, so erhält der Benutzer oder Betreiber, z.B. ein Anwender, Therapeut wie etwa ein Arzt oder eine Krankenschwester, eine Information, wie sich der Blutdruck innerhalb der nächsten Zeit, z.B. der nächsten Minuten bzw. Stunden mit den vorgegebenen Parametern, z.B. der UF-Rate, verhalten wird. Ist der vorhergesagte Blutdruck-Verlauf kritisch, so dass eine mögliche hypotensive Episode auftreten kann, besteht bereits vorab die Möglichkeit, diese zu beseitigen, indem z.B. eine automatische oder manuelle Reduktion der UF-Rate stattfindet.

Bei einem, mehreren oder allen Ausführungsbeispielen ist eine Vorhersage des intradialytischen Blutdrucks (BD) anhand patienteneigener, gespeicherter physiologischer Parameter möglich, was eine vorzeitige Regelung des Blutdrucks und somit eine Vermeidung von abfallenden Blutdruck-Trends und eine Reduktion von hypotensiven Episoden ermöglicht.

Es besteht außerdem die Möglichkeit, dem Arzt oder dem Personal einen Trend über den Verlauf des gewünschten Parameters z.B. optisch darzustellen, so dass die Interpretation des Trends erst durch das Personal geschieht, bevor ein automatischer Eingriff durch die Maschine stattfindet. Das Personal kann beispielsweise bei einem kritischen Trend rechtzeitig andere Maßnahmen ergreifen, wie z.B. eine rechtzeitige Injektion einer isotonischen Kochsalzlösung.

Alternativ kann die Vorhersage als Input in ein Biofeedbacksystem verwendet werden, welches automatisch Gegenmaßnahmen, z.B. in Form einer UF-Reduktion, einer LF-Änderung, einer Dialysierflüssigkeitstemperatur-Änderung oder einer Injektion einer isotonischen Lösung trifft.

Die Erfindung wird nachstehend anhand von Ausführungsbeispielen unter Bezugnahme auf die Zeichnungen näher beschrieben. Es zeigen:

Figur 1 ein Ablaufschema zur Vorhersage des Blutdrucks,

Figur 2 den Verlauf einer UF-Regelung,

Figur 3 eine Möglichkeit einer prospektiven UF-Regelung,

Figur 4 eine vereinfachte Darstellung eines künstlichen neuronalen Netzes,

Figur 5 den Verlauf einer Blutdruck-Vorhersage durch unterschiedliche Netzwerkmodelle,

Figur 6 eine Blutdruck-Vorhersage durch ein Netzwerk mit unterschiedlichen Trainingsalgorithmen,

Figur 7 den Verlauf einer Blutdruck-Vorhersage mit einem NAR-Network,

Figur 8 eine Blutdruck-Vorhersage mit einem NARX-Network,

Figur 9 Korrelationskoeffizienten zwischen hämodynamischen und von den Maschinensensoren aufgenommenen Parametern mit dem systolischen Blutdruck,

Figur 10 eine Blutdruck-Vorhersage mit und ohne Zeitverzögerung bei verschiedenen Lernalgorithmen,

Figur 11 eine Vorhersage des Blutdrucks bei einer Therapie durch Bildung von Ensembles, und

Figur 12 eine kontinuierliche Vorhersage einer gesamten Therapie mit einem FF-NN.

Im Folgenden werden Ausführungsbeispiele näher erläutert.

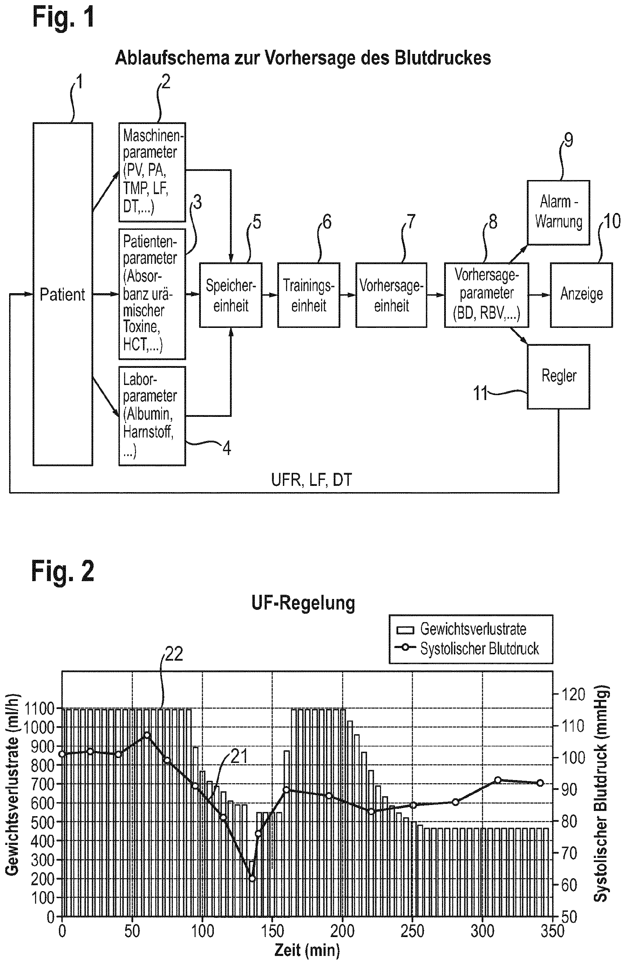

In Fig. 1 ist ein Ausführungsbeispiel der erfindungsgemäßen Vorrichtung dargestellt. Das Ausführungsbeispiel weist eine Sensor- oder Speicheranordnung 2 zur Erfassung oder Speicherung von Maschinenparametern wie etwa dem venösen Druck PV, dem arteriellen Druck PA, der Transmembrandruck TMP, der Leitfähigkeit LF der Dialysierflüssigkeit, der Dialysierflüssigkeitstemperatur DT und / oder weiterer Parameter auf. Diese Maschinenparameter können direkt durch die Sensoranordnung 2 am Patienten 1 oder an der Dialysemaschine gemessen oder aber auch bereits vorab gespeichert sein. Die von der Sensoranordnung oder Speicheranordnung 2 erfassten oder abgegebenen Werte können in einer Speichereinheit 5 gespeichert werden.

Weiterhin ist eine Sensoranordnung oder Speicheranordnung 3 zur Erfassung von Patientenparametern wie etwa der Absorbanz von urämischen Toxinen, des Hämatokrit HCT usw. vorgesehen, die ihre Eingangsgrößen durch entsprechende Messung am Patienten oder an der Dialysierflüssigkeit bzw. der Dialysemaschine erhalten kann.

Weiterhin ist eine Sensoranordnung bzw. Speicheranordnung 4 zur Erfassung oder Speicherung von Laborparametern wie etwa von Albumin, Harnstoff usw. vorgesehen, die ihre Eingangswerte vom Patienten 1 durch entsprechende Messungen oder Eingaben erhalten kann. Ebenso wie die Sensoranordnung 2 speichern auch die Sensoranordnungen 3 und 4 ihre Ausgangssignale in der Speichereinheit 5.

Auf der Basis der in der Speichereinheit 5 zwischengespeicherten Werte wie etwa der Maschinenparameter, Patientenparameter und Laborparameter wird eine der Speichereinheit 5 nachgeschaltete Trainingseinheit 6 trainiert. Diese Trainingseinheit 6 führt zunächst eine Lernphase durch, wonach sie die entsprechend trainierten, d.h. entsprechend angepassten Werte des Trainingsalgorithmus an eine Vorhersageeinheit 7 abgibt.

Die Vorhersageeinheit 7 führt durch den integrierten Vorhersagealgorithmus eine Prädiktion für die Vorhersage des zukünftigen Verlaufs von zu ermittelnden oder überwachenden Parametern durch. Diese Berechnung des zukünftigen, zu erwartenden Verlaufs der vorherzusagenden Parameter, im Folgenden auch als Vorhersageparameter bezeichnet, findet in der Vorhersageeinheit 7 statt, die beispielsweise den Blutdruck und / oder das relative Blutvolumen oder sonstige Parameter hinsichtlich ihres zu erwartenden zukünftigen Verlaufs größenmäßig ermittelt und festlegt. Die Vorhersageparameter können im Block 8 oder in mit dem Block 8 verbundenen weiteren Blöcken 9 bis 11 ausgewertet, beispielsweise mit Schwellwerten verglichen und / oder zur Anzeige gebracht werden.

Bei dem in Fig. 1 gezeigten Ausführungsbeispiel ist der Block 9 als Alarmgeber ausgelegt, der eine Warnung z.B. in akustischer und / oder optischer Form ausgibt. Hierdurch kann beispielsweise vom Arzt oder Personal ein rascher Eingriff wie etwa eine Drosselung der UF-Rate oder die Zufuhr von Flüssigkeit zu dem Patienten vorgenommen werden, bevor eine Notsituation wie etwa eine hypotensive Episode überhaupt eintritt.

Weiterhin kann der Block 10 beispielsweise als Anzeige ausgelegt sein, auf der der aktuelle Verlauf der Vorhersageparameter einschließlich des zukünftig zu erwartenden, geschätzten Verlaufs dargestellt wird.

Der Block 11 kann als Regler ausgelegt sein, der auf der Basis der Vorhersageparameter sowie auf der Basis der aktuellen Parameter, beispielsweise des Blutdrucks oder des relativen Blutvolumens, entsprechende Regeleingriffe vornimmt und damit die Behandlung des Patienten 1 entsprechend steuert.

Die Komponenten 6, 7, 8 können als künstliches neuronales Netz ausgelegt sein.

Bei einem, mehreren oder allen Ausführungsbeispielen werden Patientendaten erfasst. Hierzu werden sukzessive Blutdruck-Verläufe von Patienten über einen großen Zeitraum gesammelt, wobei mindestens 10 aufeinander folgende Therapien eines jeden Patienten zum Training eingesetzt werden. Pro Therapie werden 48 berechnete bzw. gemessene Blutdruck-Messwerte gewonnen. Für den Trainings-Prozess stehen damit 48*10 = 480 Blutdruck-Messungen desselben Patienten zur Verfügung.

Künstliche neuronale Netze werden bei Ausführungsbeispielen zur Vorhersage des Blutdrucks eingesetzt. Es können auch andere Vorhersage-Verfahren eingesetzt werden, wie z.B. Support Vector Machines. Verschiedene Netzwerktypen und -modelle werden modifiziert und getunt, so dass over- bzw. underfitting vermieden werden kann. Zur besseren Validierung der Güte der Netzwerke wird mehr als ein Kriterium betrachtet, da die alleinige Betrachtung nur eines Kriteriums, wie z.B. der Wurzel aus dem mittleren quadratischen Fehler, Root Mean Square Error (RMSE), oder des mittleren quadratischen Fehlers, Mean Square Error (MSE), zu einer falschen Validierung des Netzwerkes bzw. der Vorhersage führen könnte. Dies wurde bei mehreren Simulationen festgestellt. Der RMSE ist die Quadratwurzel des durchschnittlichen Prognosefehlers, d.h. der durchschnittlichen Abweichung der Prognose von der tatsächlichen Beobachtung. MSE bezeichnet die mittlere quadratische Abweichung eines Schätzers von dem zu schätzenden Wert.

Ausführungsbeispiele der Erfindung beschäftigen sich mit der Vorhersage intradialytischer Parameter, wie z.B. dem Blutdruck. Die Vorhersage kann mit Hilfe von Lernalgorithmen, wie z.B. mit den neuronalen Netzen, stattfinden. Diese Vorhersage ermöglicht dem Personal einen Blick über das Verhalten der Patientenparameter in die Zukunft, so dass frühzeitige Maßnahmen ergriffen werden können, oder ein rechtzeitiger automatischer Eingriff durch die Maschine erfolgt.

Da intradialytische Morbiditäten patientenspezifisch sind, ist es von Vorteil, die hämodynamischen Eigenschaften des Patienten, also z.B. den Verlauf von mindestens einem, mehreren, in beliebiger Kombination, oder allen der folgenden Parameter wie des BD, des RBV, der DT, des HCT, des PA, des PV, des TMP und / oder der Sauerstoffsättigung, zu kennen. Aus diesem Grund ist mit einem, mehreren oder allen Ausführungsbeispielen der Erfindung ein System geschaffen, das diese hämodynamischen Eigenschaften, hier auch als Hämodynamik bezeichnet, kontinuierlich lernt. Eine solche Hämodynamik kann nach jeder Therapie gespeichert werden.

Aus den gespeicherten Daten, die die Hämodynamik des Patienten in typischen und Ausnahmesituationen beschreiben, können mit Hilfe von künstlichen neuronalen Netzen Vorhersagen über den Verlauf der Hämodynamik während der laufenden Therapie getroffen werden. Künstliche neuronale Netze bieten Möglichkeiten, die dynamischen Systemveränderungen zu interpretieren und ihr zukünftiges Verhalten vorherzusagen.

Sind die hämodynamischen und physiologischen Parameter des Patienten gespeichert, kann anhand dessen ihr zukünftiger Verlauf durch das oder die neuronalen Netze vorhergesagt werden.

Lernen die künstlichen neuronalen Netze die hämodynamischen Eigenschaften des Patienten, so ist eine Vorhersage dieser Eigenschaft, abhängig von mehreren Parametern, möglich. Ist diese Vorhersage erfolgreich, so können diese Parameter rechtzeitig geregelt werden. Diese Parameter können z.B. der BD und / oder das RBV sein. Ein Alarm oder eine Warnung kann auch als Alternative oder zusätzlich ausgelöst werden. Ein manueller oder automatischer medikamentöser Eingriff kann eine Möglichkeit zur Blutdruck-Stabilisierung sein.

Bei einem, mehreren oder allen Ausführungsbeispielen ist die Vorhersage des Blutdrucks angestrebt. Der erwartete Verlauf des Blutdrucks kann innerhalb einer Dialysebehandlung mit Hilfe gespeicherter Parameter vorhergesagt werden. Besteht eine Gefahr von Hypotension, so kann das System rechtzeitig reagieren und die UF-Rate anpassen, bevor es zu Hypotonien kommt. Eine Reduktion der UF-Rate führt im Normalfall zu einer Stabilisierung des Blutdrucks und in Folge dessen der hämodynamischen Parameter. Alternativ oder zusätzlich zur Stabilisierung des Blutdrucks kann die Stabilisierung der Vorhersage einer Änderung der LF oder der DT erfolgen.

Ausführungsbeispiele der Erfindung erlauben es, z.B. intradialytische Verläufe von Patientenparametern vorherzusagen (z.B. BD). Es ist eine weitere Reduktion von Blutdruck-Messungen erreichbar. Auf etliche oder alle Blutdruck-Messungen kann verzichtet werden. Es ist eine frühzeitige Erkennung/Vorhersage von intradialytischen Morbiditäten (z.B. hypotensive Episoden) möglich. Weiterhin ist eine frühzeitige Erkennung /Vorhersage eines Blutdruck-Trends möglich. Ferner ist eine frühzeitige Interaktion im Falle von abnormalen, vorhergesagten Kurvenverläufen möglich.

Alle Blutdruck-relevanten Dialyseparameter zur Vorhersage eines anderen Dialyseparameters, z.B. des Blutdrucks werden nach jeder Dialysetherapie gespeichert. Ab einer gewissen Anzahl an gespeicherten Therapien, greift ein vordefiniertes, hinterlegtes neuronales Netz auf die vorgewählten Parameter zu. Die Parameter werden trainiert und es werden Gewichte durch das neuronale Netz berechnet. Bei einer neuen Behandlung kann durch das neuronale Netz direkt nach dem Konnektieren des Patienten und dem Einstellen seiner Parameter ein zu erwartender Blutdruck-Verlauf berechnet werden. Die Blutdruck-Verläufe werden in 5-Minuten-Intervallen vorgegeben.

Bei einem, mehreren oder allen Ausführungsbeispielen können unterschiedliche Möglichkeiten der Regelung vorgesehen sein, z.B. retrospektiv, wobei nach dem Start der Dialyse Blutdruck-Messungen in vordefinierten Zeitabständen durchgeführt werden. In der Zeit, in der keine Blutdrücke gemessen werden, werden die Blutdrücke, die durch das Netz berechnet worden sind, als aktuelle Blutdrücke genommen. Die aus dem neuronalen Netz berechneten Blutdrücke können durch ihren Trend an den aktuellen Blutdruck-Trend angepasst werden. Die Steuerung der UF-Rate orientiert sich an dem Blutdruck-Verlauf und nach dem aktuellen Blutdruck bis zur jetzigen Therapiezeit.

Eine andere Form der Regelung kann prospektiv erfolgen, wobei am Beginn der Therapiezeit, d.h. nach der Einstellung der Patientenparameter, ein Blutdruck-Verlauf für den entsprechenden Patienten berechnet wird. Es werden auch, wie gewohnt, Blutdruck-Messungen in vordefinierten Zeitabständen durchgeführt. Der Blutdruck-Verlauf wird bis zum aktuellen Zeitpunkt bewertet. Bei der prospektiven Regelung wird zusätzlich das zukünftige Verhalten des Blutdrucks, das aus der am Anfang der Therapie spezifisch für diesen Patienten berechneten Blutdruck-Kurve geschätzt wird, in Betracht gezogen und parallel bewertet. Deutet eine der beiden Bewertungen auf einen Blutdruck-Abfall oder auf einen abfallenden Blutdruck-Trend hin, kann die UF-Rate schon vor dem Auftreten dieses Blutdruck-Abfalls oder des sinkenden Blutdruck-Trends zur Stabilisierung des Blutdrucks gesenkt werden.

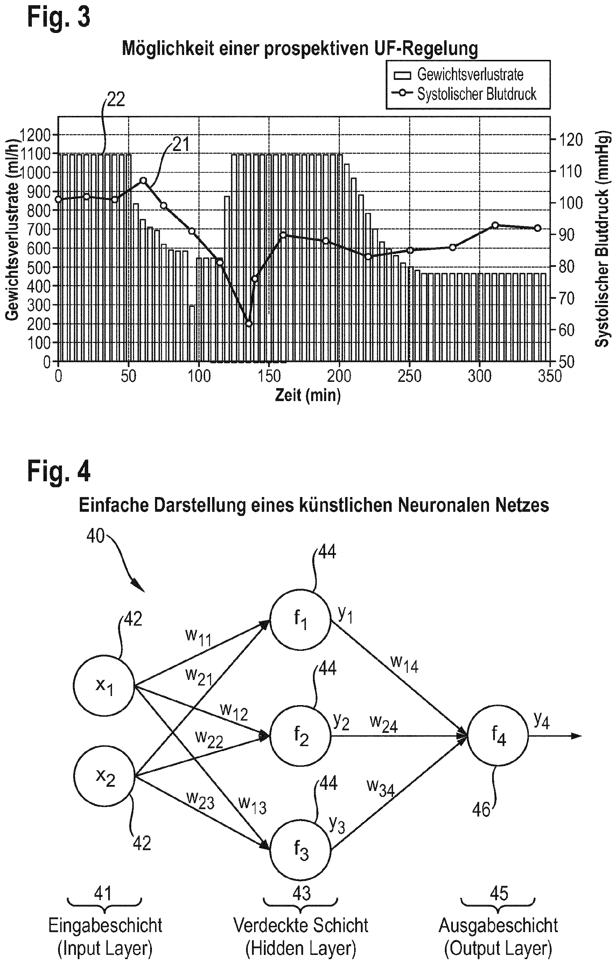

Die Zeit für die prospektive Regelung kann z.B. zwischen 5 und 60 Minuten betragen. Eine Veranschaulichung der prospektiven Regelung ist in Figur 2 und Figur 3 dargestellt. Fig. 2 zeigt eine retrospektive Regelung des Blutdrucks, indem die UF-Rate erst bei einem Erreichen einer kritischen Grenze reduziert wird.

In Fig. 2 ist mit dem Kurvenzug 21 der Verlauf des systolischen Blutdrucks in mmHg aufgetragen, wobei die runden Punkte die tatsächlichen Messwerte wiedergeben, die beispielsweise in Abständen von 20 Minuten ermittelt werden.

Auf der horizontalen Achse ist die Zeit in Minuten aufgetragen, während auf der rechten vertikalen Achse der systolische Blutdruck in seiner aktuellen Größe ausgehend von 50 bis 120 mmHg aufgetragen ist. Auf der linken vertikalen Achse ist die UF-Rate in ml/h angegeben, wobei die jeweilige Balkenlänge die jeweilige UF-Rate ("weight loss rate") angibt. Eine Maximalbeschränkung ist bei 1100 ml/h vorgegeben. Aus Fig. 2 ist ersichtlich, dass die UF-Rate bei abfallendem Blutdruck verzögert, also retrospektiv, abgesenkt wird.

In Fig. 3 ist ein Beispiel einer prospektiven UF-Anpassung der UF-Rate in Abhängigkeit von dem vorhergesagten Blutdruckverlauf bei einem Ausführungsbeispiel der Erfindung dargestellt. Analog dem Schaubild gemäß Fig. 2 ist auch bei der Darstellung gemäß Fig. 3 der Verlauf der UF-Rate von dem Blutdruckverlauf abhängig. Allerdings wird die UF-Rate beim Ausführungsbeispiel gemäß Fig. 3 bereits abgesenkt, bevor der systolische Blutdruck tatsächlich stark abfällt. Hierdurch wird eine erhöhte Patientensicherheit erreicht. Ebenso ist aus Fig. 3 ersichtlich, dass auch ein nachfolgender Wiederanstieg des Blutdrucks prospektiv vorhergesagt wird und die UF-Rate wieder hochgesetzt wird, bevor der Blutdruck tatsächlich wieder ansteigt.

In der prospektiven Regelung in Figur 3 wird der Verlauf des Blutdrucks durch die neuronalen Netze 6 bis 8 detektiert und die UF-Rate wird frühzeitig so geregelt, dass ein starker Abfall des Blutdrucks vermeidbar wird.

Die Berechnung der prospektiven Vorhersage erfolgt vor einer Therapie, z.B. während des Vorbereitens einer Dialysetherapie, unter Rückgriff auf gespeicherte Therapieverläufe. Dazu ist ein für den Patienten personalisiertes Speichermedium, wie eine Patientenkarte oder ein eindeutig abgrenzbarer Speicherbereich auf einem internen oder externen Datenträger, oder in einem externen Speicher (Datenbank), mit dem die Dialysemaschine vernetzt ist, vorhanden. Während dieser Phase kann die Struktur des Netzwerks an die Daten angepasst werden, d.h. nicht nur die Gewichte eines vordefinierten Netzwerks werden berechnet, sondern auch die Struktur des Netzwerks wird angepasst, z.B. Anzahl der Neuronen oder der Hidden Layers, um ein optimales Lernen mit dem vorhandenen Datensatz zu gewährleisten. Ebenso kann der Lernalgorithmus angepasst werden, um optimale Ergebnisse zu erhalten. Dieser Schritt ist rechenintensiv, da er die Erstellung vieler Vorhersagen umfasst, und muss zu einer Zeit durchgeführt werden, zu der die Maschine keine therapie- oder patientenkritischen Funktionen durchführt. Dies ist z.B. während der Vorbereitung der Dialysetherapie, oder auch nach Abschluss der Dialysetherapie, während der Desinfektion der Dialysemaschine, möglich.

Während der Dialysetherapie wird dann dieses ermittelte neuronale Netz verwendet, um auch neue, erst während der Dialysetherapie aufgenommene Messwerte zu bewerten und neben der vorab gerechneten Vorhersage der vollständigen Therapie in die Regelung einzubeziehen.

Bei einem, mehreren oder allen Ausführungsbeispielen ist das Lernen von intradialytischen Patientenparametern vorgesehen. Eine Früherkennung von intradialytischen Morbiditäten kann für den Patienten von großer Bedeutung sein, da dies unter anderem einen besseren Wohlzustand gewährleistet. Dieser Wohlzustand kann durch eine frühzeitige Reaktion auf das zukünftige Verhalten des gelernten Parameters (wie z.B. BD) mittels manueller (durch das Personal) oder automatischer (durch die Dialysemaschine) Intervention (z.B. Änderung der UF-Rate) gewährleistet werden.

Ein künstliches neuronales Netz besteht im allgemeinen aus Inputneuronen, Hiddenschichten und ihren Aktivierungsfunktionen und Outputneuronen.

Figur 4 stellt ein künstliches neuronales Netz 40 mit einer Eingangsschicht 41 mit mindestens zwei Eingängen (Inputs) 42 x1, x2, mindestens einer verborgenen Schicht (Hiddenschicht) 43 mit mindestens drei Neuronen 44 $f_1$, $f_2$, $f_3$, und mindestens einer Ausgangsschicht 45 mit mindestens einem Ausgang (Output) 46, $f_4$, dar. Die Ausgänge $y_1$, $y_2$, $y_3$ der Neuronen 44 bzw. 46 und der Ausgang $y_4$ mit den Aktivierungsfunktionen $f_1$, $f_2$, $f_3$ und $f_4$ werden folgendermaßen berechnet:

$$y_1 = f_1(w_{11}{}^*x_1 + w_{21}{}^*x_2)$$

$$y_2 = f_2(w_{12}{}^*x_1 + w_{22}{}^*x_2)$$

$$y_3 = f_3(w_{13}{}^*x_1 + w_{23}{}^*x_2)$$

$$y_4 = f_4(w_{14}{}^*y_1 + w_{24}{}^*y_2 + w_{34}{}^*y_3)$$

Hierbei stellen $f_1$, $f_2$, $f_3$ und $f_4$ die Aktivierungsfunktionen dar. Die Aktivierungsfunktionen können übliche mathematische Funktionen, wie z.B. Sigmoidfunktion, Schwellenwertfunktion, etc, sein.

Zur Erzeugung des Outputs (Ausgangssignals) $y_4$ werden die drei Neuronen-Ausgangsgrößen $y_1$, $y_2$, $y_3$ nach Bewertung, hier Multiplikation, mit Gewichtungen $w_{14}$, $w_{24}$, $w_{34}$ verrechnet, hier z.B. addiert.

Es wurden mehrere Netzwerktypen mit unterschiedlicher Anzahl von Hiddenschichten 43 und unterschiedlicher Anzahl von Neuronen 44 getestet. Das Ändern des Netzwerkmodells unter einem und dem gleichen Lernalgorithmus führt zu unterschiedlichen Vorhersagen. Es bestehen also unendliche Kombinationen zwischen Netzwerkmodellen, die zu unterschiedlichen Ergebnissen führen.

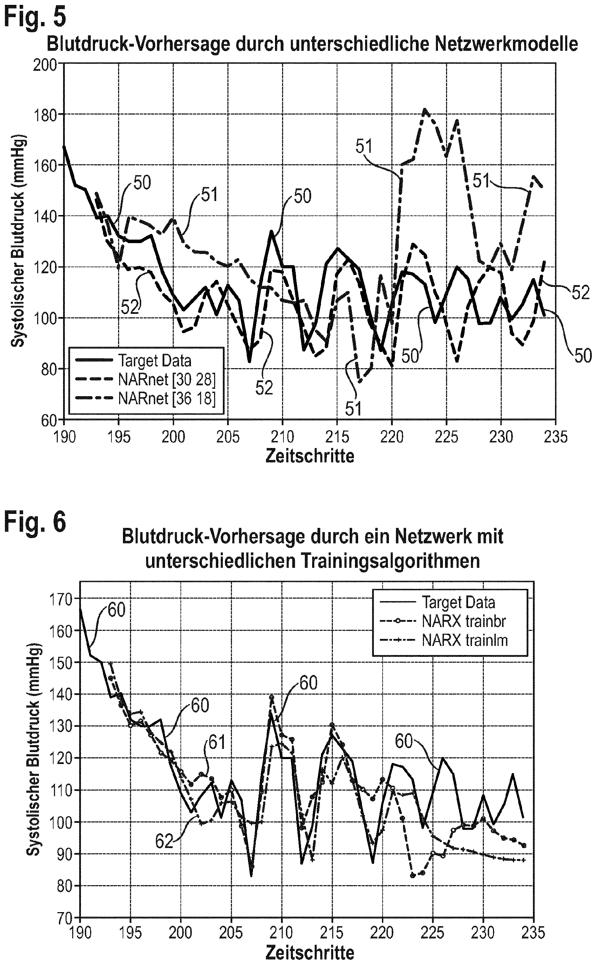

Figur 5 zeigt das Ergebnis von zwei mit dem gleichen Trainingsalgorithmus, aber mit unterschiedlichen Netzwerkstrukturen trainierten Netzwerke. Beide Netzwerke wurden mit dem *Levenberg-Marquardt-Backpropagation-Algorithmus* trainiert. Die Kurve 50 zeigt den tatsächlichen Verlauf des Blutdrucks. Die Kurve 52 ist das Ergebnis der Vorhersage eines Netzwerkes mit zwei Hiddenschichten, wobei die erste aus 30 und die zweite aus 28 Neuronen besteht. Die Kurve 51 spiegelt die Vorhersage mit Hilfe eines Netzwerks wider, das ebenso aus zwei Hiddenschichten besteht, wobei die erste aus 36 und die zweite aus 18 Neuronen besteht. Figur 5 zeigt also eine Blutdruck-Vorhersage mit unterschiedlichen Netzwerkmodellen.

Ein anderer Faktor, der zu unterschiedlichen Vorhersagen führen kann, sind die unterschiedlichen Trainingsalgorithmen. Das Training der Netzwerke erfolgte unter anderem mit dem *Bayesian Regulation-Backpropagation-* und mit dem *Levenberg-Marquardt-Backpropagation-Algorithmus.* Die Evaluation der Performance des Outputs, die Vorhersage des Blutdrucks, wurde mit Hilfe statistischer Mittel, wie z.B. Root Mean Square Error (RMSE) und Korrelation, etc. und mit Visualisierungskriterien bewertet.

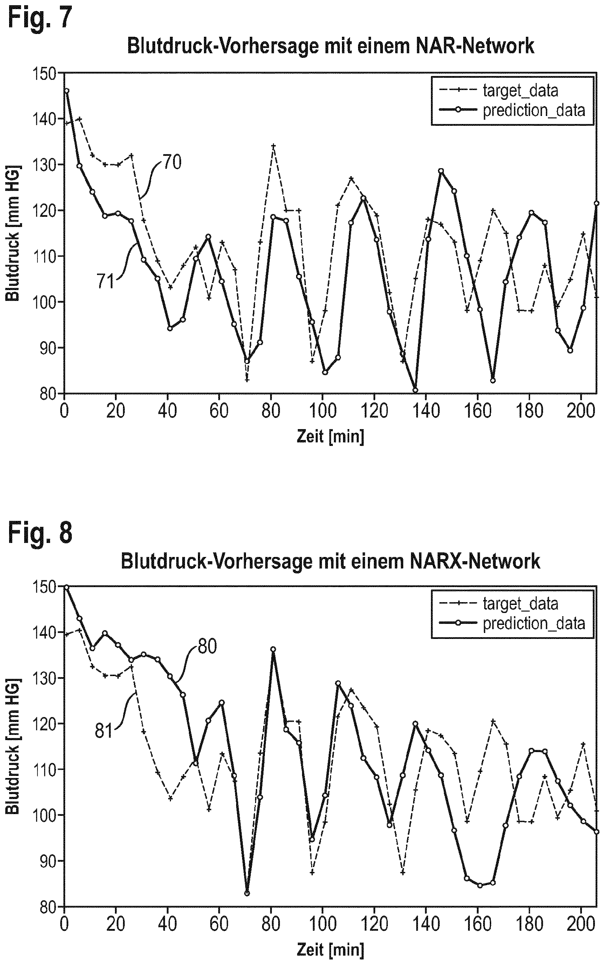

Die Figuren 7, 8 stellen Abbildungen der Verläufe der Vorhersage mit dem Nonlinear Autoregressive (NAR)-Network und mit dem Nonlinear Autoregressive Exogenous (NARX)-Network dar. Bei NARX Networks werden mehrere Input und Output Parameter erwartet.

Die Kurven 70 bzw. 80 zeigen den tatsächlichen Verlauf des Blutdrucks. Figur 7 zeigt eine BD-Vorhersage 71 mit einem NAR-Network, während in Figur 8 eine Blutdruck-Vorhersage 81 mit einem NARX-Network gezeigt ist.

Aus den Figuren 7 und 8 ist zu erkennen, dass das Training mit dem NARX Network (Figur 8) zu besseren Ergebnissen am Anfang der Therapie führt, wobei das NAR-Network (Figur 7) eine gewisse Verschiebung aufweist.

Figur 6 zeigt eine BD-Vorhersage durch ein Netzwerk mit unterschiedlichen Trainingsalgorithmen. Die Kurve 60 zeigt den tatsächlichen Verlauf des Blutdrucks. Figur 6 stellt die Vorhersage des Blutdrucks mit beider Trainingsalgorithmen dar: des *Bayesian-Regulation-Backpropagation,* veranschaulicht durch die Kurve 61 mit runden Kreisen, und des *Levenberg-Marquardt-Backpropagation-Algorithmus,* veranschaulicht durch die Kurve 62 mit den Plus-Zeichen.

Andere Netzwerkmodelle bieten die Möglichkeit, verschiedene Inputparameter zu nehmen. Solche Netze werden als Nonlinear Autoregressive Network Exogenous (NARX) bezeichnet. Für diese Art Netzwerk wurden bei dem dargestellten Ausführungsbeispiel zwei Inputparameter gewählt: der BD und die UF-Rate. Es können auch mehrere oder andere Inputparameter gewählt werden.

Aus den Daten ist klar zu erkennen, dass die Vorhersage von BD-Werten auf Basis neuronaler Netze gut für die erste Hälfte der Therapie funktioniert, in der zweiten Hälfte aber Schwächen aufweist. Dort steigen die Abweichungen zwischen Vorhersage und gemessenem BD an. Eine Verbesserung der Vorhersagequalität wird bei einem, mehreren oder allen Ausführungsbeispielen dadurch erzielt, dass während der Therapie gemessene Blutdrücke in eine während der Therapie durchgeführte Bewertung und Neuberechnung der Vorhersage einbezogen werden. Diese findet auf Basis des vorab definierten Netzwerks statt.

Bei einem, mehreren oder allen Ausführungsbeispielen kann vorgesehen sein, zur weiteren Verbesserung der Vorhersage weitere Inputparameter und weitere Lerntechniken einzubeziehen.

Das bei einem oder mehreren Ausführungsbeispielen weiter vorgesehene Einbeziehen der UF-Rate neben dem Blutdruck als Inputparameter führt zu einer noch besseren Vorhersage des Blutdrucks. Aus diesem Grund sind mehrere hämodynamische und von der Maschine gemessene Parameter in Betracht genommen. Diese wurden zuerst auf Korrelation mit dem Blutdruck untersucht.

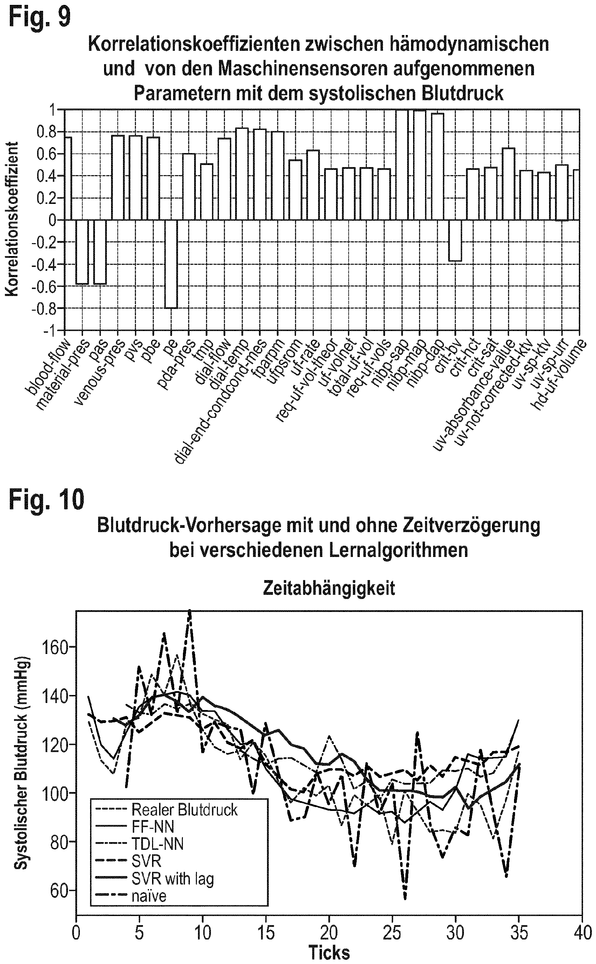

Figur 9 zeigt Korrelationskoeffizienten zwischen hämodynamischen und von den Maschinensensoren aufgenommenen Parameter mit dem systolischen Blutdruck. Wie aus dieser Abbildung zu erkennen, wurden 30 Dialyse- und Patientenparameter auf Korrelation mit dem Blutdruck geprüft. Zahlreiche Parameter hatten einen Korrelationskoeffizieten höher als 0,5. Manche dieser Parameter erreichten einen Korrelationskoeffizieten von etwa 0,8, wobei diese Parameter in keinem Verhältnis zu dem Blutdruck stehen. Dieser Effekt trat aufgrund des Verlaufes dieser Parameter auf. Der Verlauf war nahezu konstant. Aus diesem Korrelationsverfahren wurden die Parameter ausgesucht, die gewissermaßen eine hohe Korrelation mit dem Blutdruck aufweisen.

Es wurden danach verschiedene Merkmalsselektions- (Feature Selection-) Algorithmen an mehreren Parametern verwendet. Diese Algorithmen können z.B. der *minimum-Redundancy-maximum-Relevance* (mRmR) Algorithmus sein, der gemeinsame Informationskriterien zwischen Blutdruck und anderen Parameter nutzt, um ein gewisses Ranking der Variablen zu bilden, oder der *Wrapper* Algorithmus, der auf maschinellem Lernen basiert ist, der durch Aufaddieren oder Löschen von weiteren Variablen versucht, eine beste Performance zu finden, die durch statistische Mittel, wie z.B. mittlere Fehlerquadrate, evaluiert wird.

Nach Expertenwissen, Datenanalyse und Feature Selection-Algorithmen wurden mehrere Inputvariablen für das Trainieren des Netzes gewählt, wie z.B. einer, mehrere (in beliebigen Kombinationen) oder alle der folgenden Parameter:

das Ultrafiltrationsvolumen,
der arterielle und venöse Druck,
der Hämatokrit,
das relative Blutvolumen,
die Sauerstoffsättigung,
der Hämoglobin,
die Ultrafiltrationsrate,
die Herzrate,

die Absorbanz urämischer Toxine,
der Systolische Blutdruck,
die Dialysierflüssigkeitsleitfähigkeit (Säure und Base Leitfähigkeit, PH-Wert),
die Dialysierflüssigkeitstemperatur.

Als nächstes wurden im allgemeinen zwei Maschinelles-Lernen-Algorithmen und ein naiver Algorithmus evaluiert, wobei die neuronalen Netze mit dem Feed Forward Neural Network (FF-NN) mit und ohne Tapped Delayed Lines (Zeitabhängigkeit (TDL-NN)), die Support Vector Regression (SVR) mit und ohne Lag (Überlappung) und ein naiver Algorithmus, der die vorhandenen Blutdruck-Werte auf den nächsten Wert extrapoliert, eingesetzt wurden. Bei dem Training wurde eine Cross Validation durchgeführt. Diese teilt die Therapien, deren Variablen trainiert werden müssen, in unterschiedliche Blöcke ein. Es werden z.B. die ersten und letzten 10 Therapien trainiert und auf die mittleren Therapien validiert, oder auf die ersten Therapien trainiert und auf die letzten validiert. Dadurch erreicht man eine Erhöhung der Trainingsdaten. Danach wird das Netz mit der besten Performance gewählt.

Bei der Vorhersage wurde auf Zeitabhängigkeit, d.h. ob alte Variablenwerte aus der aktuellen Therapie berücksichtigt werden müssen, um den aktuellen Blutdruck vorherzusagen, auf bessere Vorhersage-Performance durch Bildung von Ensembles, d.h. durch Kombination von verschiedenen Lernalgorithmen und von verschiedenen Netzstrukturen eines Lernalgorithmus und auf kontinuierliche Vorhersage einer Therapie geprüft.

Im folgenden werden Ergebnisse der einzelnen Experimenten dargestellt:
Figur 10 zeigt die Zeitabhängigkeit und veranschaulicht die Blutdruck-Vorhersage mit und ohne Zeitverzögerung bei verschiedenen Lernalgorithmen.

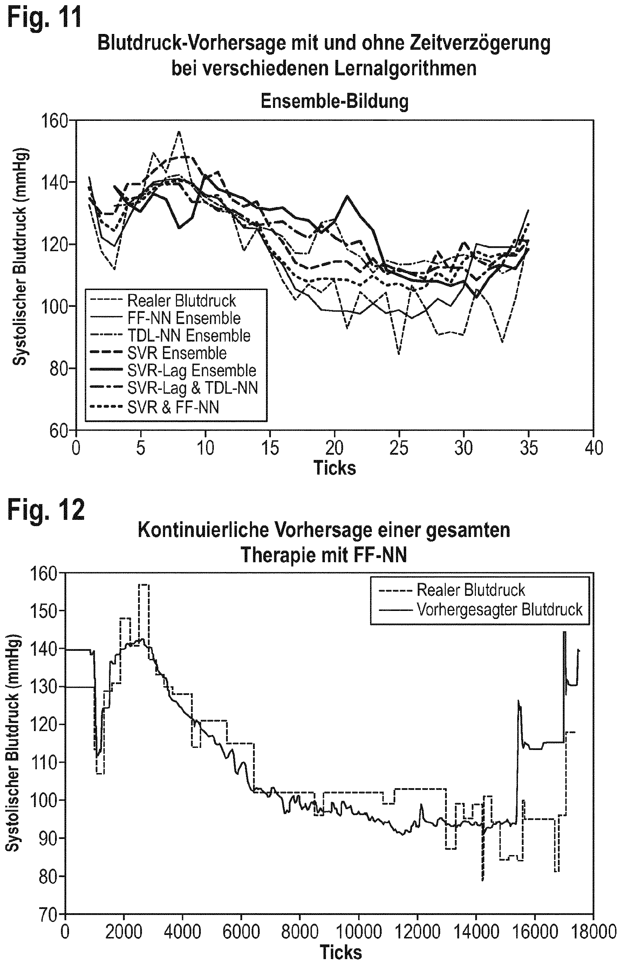

Figur 11 zeigt eine Ensemble-Bildung und veranschaulicht präziser eine Vorhersage des Blutdrucks bei einer Therapie durch Bildung von Ensembles.

Bei einem, mehreren oder allen Ausführungsbeispielen kann auch eine kontinuierliche Vorhersage einer gesamten Therapie vorgesehen sein.

Figur 12 zeigt eine kontinuierliche Vorhersage einer gesamten Therapie mit einem neuronalen Netz mit Vorwärtskopplung, FF-NN (FeedForward Neural Network).

In Figur 10 ist zu erkennen, dass das neuronale Netz mit Vorwärtskopplung (FeedForward Neural Network, FF-NN) am besten dem Kurvenverlauf folgt.

Eine gute Vorhersage wurde auch durch den naiven Algorithmus erzielt. Diese schwankt aber um den realen Blutdruck aufgrund der permanenten, linearen Extrapolation der letzten zwei gemessenen Blutdrücke zum nächsten fünf Minutenintervall herum.

Es ist aus Figur 10 und dementsprechend auch aus dem Experiment der Zeitabhängigkeit weiterhin zu schließen, dass Blutdruck-Messwerte mit dem FF-NN vorhergesagt werden können, ohne dass alte oder aktuelle, aus der aktuellen Therapie gemessene Blutdruck-Werte in die Vorhersage mit einbezogen werden.

Tabelle 1 zeigt die Ergebnisse der MSE des Experiments mit der Zeitabhängigkeit mit den verschiedenen Lernalgorithmen. FF-NN zeigt weiterhin den kleinsten MSE von 120,495. Der Naive Algorithmus zeigt den zweitbesten MSE. Dies liegt, wie oben beschrieben, an der linearen Extrapolation. Alle anderen MSEs zeigen sehr hohe Werte und somit auch schlechte Vorhersagen.

**Tabelle 1 Liste der Ergebnisse der MSE des Experiment mit der Zeitabhängigkeit**

| Patient | Naive | TDL-NN | Lagged SVR | FF-NN | SVR |
|---|---|---|---|---|---|
| 1 | 62,5 | 104,4 | 188,2 | 52,4 | 243,5 |
| 2 | 162,5 | 282,8 | 350 | 153,1 | 405,2 |
| 3 | 230,6 | 245,5 | 515 | 167,5 | 132,8 |
| 4 | 108,2 | 184,9 | 425,5 | 122,1 | 267,5 |
| 5 | 148,6 | 319,8 | 325 | 119,5 | 210,1 |
| 6 | 172,5 | 167,7 | 883 | 108,2 | 172,8 |
| Mean | 147,5 | 217,5 | 447,8 | 120,5 | 238,6 |

In Figur 11 ist zu erkennen, dass fast jede Ensemble-Bildung dem Verlauf des Blutdrucks folgt, abgesehen von dem SVR-Lag Ensemble. Es ist festzustellen, dass das FF-NN Ensemble und das SVR & FF-NN Ensemble die beiden Kurven sind, die am genauesten dem Blutdruck folgen.

In Figur 12 ist eine kontinuierliche Vorhersage einer Therapie dargestellt, bei der jede Sekunde ein Blutdruck vorhergesagt wird.

Bei allen Evaluierungen wurden auch statistische Mittel, wie z.B. das MSE, das Hit Ratio, das die Streuung oder Abweichung eines vorhergesagten Blutdrucks gegenüber dem ursprünglichen Blutdruck beschreibt, und statistische Algorithmen, wie z.B. das Aikake Information Criteria (AIC) und das Bayesian Information Criteria (BIC) eingesetzt, die die Performance der Vorhersage bewerten.

Die Ausführungsbeispiele und Figuren stellen die bisher erzielten Ergebnisse zur Vorhersage von Blutdrücken unter Einbeziehung von Blutdruck-Messungen und UF-Raten vergangener Therapien dar. Jedoch ist die Erfindung nicht hierauf eingeschränkt.

Prinzipiell ist die Vorhersage anderer Parameter des Patienten, z.B. Hämatokrit, relatives Blutvolumen, Sauerstoffsättigung, usw., mit ähnlicher Technik möglich.

Die Anzahl der Inputparameter in das neuronale Netzwerk ist ebenfalls nicht beschränkt auf die hier verwendeten Parameter, und kann neben früheren Blutdruck-Werten und UF-Raten auch andere Parameter, wie Hämatokrit, relatives Blutvolumen, Sauerstoffsättigung, etc. mit einbeziehen.

**ABKÜRZUNGEN** *Abbreviations*

| | |
|---|---|
| AIC | Aikake Information Criteria |
| BD | Blutdruck |
| BIC | Bayesian Information Criteria |
| br | Bayesian regulation backpropagation |
| DT | Dialysierflüssigkeitstemperatur |
| FF | Feed Forward, Vorwärtskopplung |
| HCT | Hämatocrit |
| lm | Levenberg-Marquardt backpropagation |
| mRmR | Minimum-Redundancy-Maximum-Relevance |
| NAR | Nonlinear Autoregressiv Neural Network |
| NARX | Nonlinear Autoregressiv Neural Network Exogenous |
| NN | Neuronales Netz |
| PA | Arterieller Druck |
| PV | Venöser Druck |
| RBV | Relatives Blutvolumen |
| SO2 | Sauerstoffsättigung |
| SVR | Support Vector Regression, Stützvektorregression |
| TMP | Transmembrandruck |

**Patentansprüche**

1. Vorrichtung zur Vorhersage eines intradialytischen Parameters, nämlich eines Blutdrucks, und zur prospektiven Ultrafiltrations-Anpassung der Ultrafiltrationsrate, UF-Rate, in Abhängigkeit von dem vorhergesagten Blutdruckverlauf,

   wobei mindestens ein Lernalgorithmus und / oder mindestens ein neuronales Netz vorgesehen ist,

   wobei die Vorrichtung dazu ausgelegt ist, den Verlauf der UF-Rate in Abhängigkeit von dem Blutdruckverlauf derart festzulegen, dass die UF-Rate bereits abgesenkt wird, bevor der systolische Blutdruck tatsächlich stark abfällt,

   wobei die Vorrichtung weiterhin dazu ausgelegt ist, dass auch ein nachfolgender Wiederanstieg des Blutdrucks prospektiv vorhergesagt wird und die UF-Rate wieder hochgesetzt wird, bevor der Blutdruck tatsächlich stark ansteigt,

   mit einer Speichereinrichtung zur Speicherung von patientenindividuellen intradialytischen Parametern, Laborparametern und/oder Maschinenparametern, die bei der Vorhersage des Blutdrucks während einer Dialysebehandlung einsetzbar sind, und

   mit einem System, das die Vorhersage des Blutdrucks als Input verwendet und automatisch Gegenmaßnahmen in Form einer UF-Ratenveränderung trifft,

   wobei die Vorrichtung dazu ausgelegt ist, zur Verbesserung der Vorhersagequalität während der Therapie gemessene Blutdrücke in eine während der Therapie durchgeführte Bewertung und Neuberechnung der Vorhersage einzubeziehen.

2. Vorrichtung nach Anspruch 1, wobei die patientenindividuellen intradialytischen Parameter der Blutdruck und / oder das relative Blutvolumen, RBV, sind, und / oder die Laborparameter Albumin und / oder Harnstoff sind und / oder die Maschinenparameter ein venöser Druck, PV, und / oder ein arterieller Druck, PA, sind.

3. Vorrichtung nach einem der vorhergehenden Ansprüche, wobei ein Trend über den Verlauf des gewünschten Parameters angezeigt wird, z.B. optisch oder akustisch.

4. Vorrichtung nach einem der vorhergehenden Ansprüche, mit einem Biofeedbacksystem, das die Vorhersage als Input verwendet und automatisch Gegenmaßnahmen, z.B. in Form einer UF-Reduktion, einer LF-Änderung, einer Dialysierflüssigkeitstemperatur-Änderung oder einer Injektion einer isotonischen Lösung trifft.

5. Vorrichtung nach einem der vorhergehenden Ansprüche, mit mindestens einer Sensor- oder Speicheranordnung (2) zur Erfassung oder Speicherung von Maschinenparametern wie etwa einem venösen Druck PV, einem arteriellen Druck PA, einem Transmembrandruck TMP, einer Leitfähigkeit LF der Dialysierflüssigkeit, einer Dialysierflüssigkeitstemperatur DT und / oder weiterer Parameter, und / oder
einer Sensoranordnung oder Speicheranordnung (3) zur Erfassung von Patientenparametern wie etwa einer Absorbanz von urämischen Toxinen, des Hämatokrit HCT, und / oder
einer Sensoranordnung bzw. Speicheranordnung (4) zur Erfassung oder Speicherung von Laborparametern wie etwa von Albumin, Harnstoff usw.

6. Vorrichtung nach einem der vorhergehenden Ansprüche, mit einer Trainingseinheit (6) und einer Vorhersageeinheit (7), wobei die Trainingseinheit dazu ausgelegt ist, zunächst eine Lernphase durchzuführen, wonach sie die entsprechend trainierten, d.h. entsprechend angepassten Werte eines Trainingsalgorithmus an die Vorhersageeinheit (7) abgibt, die durch den integrierten Trainingsalgorithmus eine Prädiktion aufgrund von Extrapolationen oder anderweitigen Berechnungen für die Vorhersage des zukünftigen Verlaufs von zu ermittelnden oder überwachenden Parametern durchführt,
wobei die Vorhersageparameter ausgewertet, mit Schwellwerten verglichen und / oder zur Anzeige gebracht werden können.

7. Vorrichtung nach Anspruch 6, bei der die Trainingseinheit (6), die Vorhersageeinheit (7) und / oder eine Vorhersageparameter bildende Einrichtung (8) jeweils als künstliches neuronales Netz ausgelegt sind und / oder Support Vector Machines, Stützvektormaschinen, eingesetzt werden.

8. Vorrichtung nach einem der vorhergehenden Ansprüche, mit einem Alarmgeber zur Ausgabe einer Warnung z.B. in akustischer und / oder optischer Form, und / oder einer Anzeige, auf der der aktuelle Verlauf der Vorhersageparameter einschließlich des zukünftig zu erwartenden, geschätzten Verlaufs darstellbar ist.

9. Vorrichtung nach einem der vorhergehenden Ansprüche, mit einem Regler (11), der auf der Basis der Vorhersageparameter sowie auf der Basis der aktuellen Parameter, beispielsweise des Blutdrucks oder des relativen Blutvolumens, Regeleingriffe vornimmt.

10. Vorrichtung nach einem der vorhergehenden Ansprüche, die dazu ausgelegt ist,
während einer anfänglichen Phase die Struktur eines neuronalen Netzwerks (40) mit einer Eingangsschicht (41) mit mindestens zwei Eingängen, mindestens einer verborgenen Schicht (43) mit mindestens drei Neuronen (44), und mindestens einer Ausgangsschicht (45) mit mindestens einem Ausgang (46), anzupassen,
die Ausgänge an Eingangsdaten anzupassen,
Gewichte des vordefinierten Netzwerks (40) zu berechnen, und
die Struktur des Netzwerks, z.B. die Anzahl der Neuronen oder von Hidden Layers, zu verändern, um ein optimiertes Lernen mit einem vorhandenen Datensatz zu gewährleisten, und den Lernalgorithmus anzupassen.

11. Vorrichtung nach einem der vorhergehenden Ansprüche, bei der als Trainingsalgorithmen ein Training der Netzwerke mit dem *Bayesian Regulation-Backpropagation*- und mit dem *Levenberg-Marquardt-Backpropagation-Algorithmus* vorgesehen ist, und / oder Verläufe der Vorhersage mit einem Nonlinear Autoregressive (NAR)-Network und / oder mit einem Nonlinear Autoregressive Exogenous (NARX)-Network ausgewertet werden.

12. Vorrichtung nach einem der vorhergehenden Ansprüche, die dazu ausgelegt ist, zur weiteren Verbesserung der Vorhersage mindestens einen oder mehrere der folgenden Inputparameter neben dem Blutdruck,
Ultrafiltrationsrate, UF-Rate,.
Ultrafiltrationsvolumen,
arterieller und venöser Druck,
Hämatokrit,
relatives Blutvolumen,

Sauerstoffsättigung,
Hämoglobin,
Ultrafiltrationsrate,
Herzrate,
Absorbanz urämischer Toxine,
systolischer Blutdruck,
Dialysierflüssigkeitsleitfähigkeit (Säure und Base Leitfähigkeit, PH-Wert), und / oder
Dialysierflüssigkeitstemperatur, und / oder
weitere Lerntechniken einzubeziehen.

**Claims**

1. A device for predicting an intradialytic parameter, that is blood pressure, and for the prospective ultrafiltration adaptation of the ultrafiltration rate, UF rate, as a function of the predicted blood pressure progress,
   wherein at least one learning algorithm and/or at least one neural network are provided,
   the device being configured to set the progress of the UF rate as a function of the blood pressure progress such that the UF rate is already being lowered before a sharp drop in the systolic blood pressure actually occurs,
   wherein the device is furthermore configured such that a subsequent, new rise in the blood pressure is also predicted in a prospective manner and the UF rate is again increased before the blood pressure actually sharply rises,
   comprising a memory device for storing patient-individual intradialytic parameters, laboratory parameters, and/or machine parameters, which can be used in the prognosis of the blood pressure during a dialysis treatment, and comprising a system which uses the prognosis of the blood pressure as an input and automatically takes countermeasures in the form of a UF rate reduction,
   wherein the device is configured to include blood pressures which have been measured during the therapy unit in the evaluation and recalculation of the prognosis carried out during the therapy unit to improve the prognosis quality.

2. The device according to claim 1, wherein the patient-individual intradialytic parameters are the blood pressure and/or the relative blood volume RBV, and/or the laboratory parameters are albumin and/or urea, and/or the machine parameters are the venous pressure PV and/or the arterial pressure PA.

3. The device according to one of the preceding claims, wherein a trend in the progress of the desired parameter is displayed, e.g. visually or acoustically.

4. The device according to one of the preceding claims, comprising a biofeedback system which uses the prognosis as an input and automatically takes countermeasures, e.g. in the form of UF reduction, an LF change, a change in the temperature of the dialysis liquid or an injection of an isotonic solution.

5. The device according to one of the preceding claims, comprising at least one sensor or memory arrangement (2) for detecting or storing machine parameters, such as the venous pressure PV, arterial pressure PA, transmembrane pressure TMP, a conductivity LF of the dialysis liquid, temperature DT of the dialysis liquid, and/or further parameters, and/or
   a sensor arrangement or memory arrangement (3) for detecting patient parameters, such as the absorbance of uremic toxins, of the hematocrit HCT, and/or
   a sensor arrangement or memory arrangement (4) for detecting or storing laboratory parameters such as those of albumin, urea etc.

6. The device according to one of the preceding claims, comprising a training unit (6) and a prognosis unit (7), wherein the training unit is designed to carry out a learning phase first, whereupon it forwards the correspondingly trained, i.e. correspondingly adapted, values of a training algorithm to the prognosis unit (7) which by means of the integrated training algorithm performs a prediction, based on extrapolations or other calculations, for the prognosis of the future progress of parameters to be determined or monitored,
   wherein the prognosis parameters can be evaluated, compared with threshold values, and/or displayed.

7. The device according to claim 6, in which the training unit (6), the prognosis unit (7), and/or a means (8) of forming prognosis parameters are each designed as an artificial neural network and/or used as support vector machines.

8. The device according to one of the preceding claims, comprising an alarm unit for outputting a warning, e.g. in acoustic and/or visual form, and/or in the form of a display device on which the current progress of the prognosis parameters can be shown, as well as the estimated progress to be expected in future.

9. The device according to one of the preceding claims, comprising a controller (11) which takes control interventions based on the prognosis parameters as well as on the current parameters, for instance the blood pressure or the relative blood volume.

10. The device according to one of the preceding claims, which is designed to adapt, during an initial phase, the structure of a neural network (40) comprising an input layer (41) with at least two inputs, at least one hidden layer (43) comprising at least three neurons (44), and at least one output layer (45) with at least one output (46),
adapt the outputs to input data,
calculate the weights of the predefined network (40), and
alter the structure of the network, e.g. the number of the neurons or of the hidden layers, in order to ensure an optimized learning process with an existing data record, and to adapt the learning algorithm.

11. The device according to one of the preceding claims, in which the training of the networks is performed with the *Bayesian Regulation Backpropagation* and *Levenberg-Marquardt Backpropagation* algorithms as the training algorithms, and/or the progress of the prognosis is evaluated with a nonlinear autoregressive (NAR) network and/or with a nonlinear autoregressive exogenous (NARX) network.

12. The device according to one of the preceding claims, which is designed, for further improving the prognosis, to involve at least one or more of the following input parameters, in addition to the blood pressure:

> ultrafiltration rate, UF rate,
> ultrafiltration volume,
> arterial and venous pressure,
> hematocrit,
> relative blood volume,
> oxygen saturation,
> hemoglobin,
> ultrafiltration rate,
> heartbeat,
> absorbance of uremic toxins,
> systolic blood pressure,
> conductivity of the dialysis liquid (acid and base conductivity, pH value), and/or temperature of the dialysis liquid, and/or
> further learning techniques.

**Revendications**

1. Dispositif de prédiction d'un paramètre intradialytique, à savoir d'une pression artérielle, et pour l'ajustement d'ultrafiltration prospectif des taux d'ultrafiltration, taux d'UF, en fonction de l'évolution de la pression artérielle prédite, dans lequel au moins un algorithme d'apprentissage et/ou au moins un réseau neuronal est prévu,
dans lequel le dispositif est conçu pour déterminer l'évolution du taux d'UF en fonction de l'évolution de la pression artérielle de telle sorte que le taux d'UF soit déjà abaissé avant que la pression artérielle systolique diminue effectivement fortement,
dans lequel le dispositif est en outre conçu pour qu'également une remontée consécutive de la pression artérielle soit prédite de façon prospective et que le taux d'UF soit à nouveau augmenté, avant que la pression artérielle augmente effectivement fortement,
avec une installation d'enregistrement pour l'enregistrement de paramètres intradialytiques individuels du patient, des paramètres de laboratoire et/ou des paramètres de machine qui peuvent être utilisés lors de la prédiction de la pression artérielle pendant un traitement par dialyse, et
avec un système qui utilise la prédiction de la pression artérielle comme entrée et prend automatiquement des contre-mesures sous la forme d'une modification des taux d'UF,
dans lequel le dispositif est conçu, pour l'amélioration de la qualité de prédiction, pour prendre en compte des pressions artérielles mesurées pendant la thérapie dans une évaluation réalisée pendant la thérapie et un nouveau calcul de la prédiction.

**2.** Dispositif selon la revendication 1, dans lequel les paramètres intradialytiques individuels du patient sont la pression artérielle et/ou le volume sanguin relatif, RBV, et/ou les paramètres de laboratoire sont l'albumine et/ou l'urée et/ou les paramètres de machine sont une pression veineuse, PV, et/ou une pression artérielle, PA.

**3.** Dispositif selon l'une quelconque des revendications précédentes, dans lequel une tendance est indiquée sur l'évolution du paramètre souhaitée, par exemple de façon optique ou acoustique.

**4.** Dispositif selon l'une quelconque des revendications précédentes, doté d'un système de rétroaction biologique qui utilise la prédiction comme entrée et prend automatiquement des contre-mesures, par exemple sous la forme d'une réduction d'UF, d'une modification de LF, une modification de la température du fluide de dialyse ou d'une injection d'une solution isotonique.

**5.** Dispositif selon l'une quelconque des revendications précédentes, doté d'au moins un agencement de capteur ou de mémoire (2) pour la saisie ou l'enregistrement de paramètres de machine comme par exemple une pression veineuse PV, une pression artérielle PA, une pression transmembranaire TMP, une conductivité LF du fluide de dialyse, une température du fluide de dialyse DT et/ou d'autres paramètres, et/ou
un agencement de capteur ou un agencement de mémoire (3) pour la saisie de paramètres du patient comme par exemple d'une absorbance de toxines urémiques, de l'hématocrite HCT, et/ou
un agencement de capteur ou un agencement de mémoire (4) pour la saisie ou l'enregistrement de paramètres de laboratoire comme par exemple l'albumine, l'urée etc.

**6.** Dispositif selon l'une quelconque des revendications précédentes, doté d'une unité de formation (6) et d'une unité de prédiction (7), dans lequel l'unité de formation est conçue pour réaliser tout d'abord une phase d'apprentissage, après quoi elle délivre les valeurs de formation correspondante, c'est-à-dire les valeurs ajustées de façon correspondante d'un algorithme de formation à l'unité de prédiction (7) qui réalise par le biais de l'algorithme de formation intégré, une prédiction sur la base d'extrapolations ou d'autres calculs pour la prédiction de l'évolution future de paramètres à déterminer ou à surveiller,
dans lequel les paramètres de prédiction peuvent être évalués, comparés à des valeurs seuils et/ou affichés.

**7.** Dispositif selon la revendication 6, dans lequel l'unité de formation (6), l'unité de prédiction (7) et/ou une installation (8) formant des paramètres de prédiction sont conçues respectivement sous la forme d'un réseau neuronal artificiel et/ou sont utilisées comme Support Vector Machines, machines à vecteurs de support.

**8.** Dispositif selon l'une quelconque des revendications précédentes, doté d'une alarme pour l'émission d'un avertissement par exemple sous forme acoustique et/ou optique, et/ou d'un affichage, sur laquelle l'évolution actuelle des paramètres de prédiction y compris de l'évolution évaluée, attendue à l'avenir peut être représentée.

**9.** Dispositif selon l'une quelconque des revendications précédentes, doté d'un régulateur (11) qui intervient sur la base des paramètres de prédiction ainsi que sur la base des paramètres actuels, par exemple de la pression artérielle ou du volume sanguin relatif.

**10.** Dispositif selon l'une quelconque des revendications précédentes, qui est conçu pour
ajuster, pendant une phase initiale, la structure d'un réseau neuronal (40) avec une couche d'entrée (41) dotée d'au moins deux entrées, d'au moins une couche cachée (43) avec au moins trois neurones (44), et d'au moins d'une couche de sortie (45) avec au moins une sortie (46),
ajuster les sorties aux données d'entrée,
calculer des poids du réseau prédéfini (40), et
modifier la structure du réseau, par exemple le nombre de neurones ou de couches cachées, pour assurer un apprentissage optimisé avec un groupe de données présentes, et pour ajuster l'algorithme d'apprentissage.

**11.** Dispositif selon l'une quelconque des revendications précédentes, dans lequel comme algorithmes de formation, une formation des réseaux est prévue avec un algorithme de régulation-rétropropagation bayésienne et avec l'algorithme Levenberg-Marquardt de rétropropagation, et/ou des évolutions des prédictions sont évaluées avec un réseau autorégressif non linéaire (NAR) et/ou avec un réseau autorégressif exogène non linéaire (NARX).

**12.** Dispositif selon l'une quelconque des revendications précédentes, qui est conçu pour améliorer encore les prédictions d'au moins l'un ou plusieurs des paramètres d'entrée suivants en dehors de la pression artérielle, taux d'ultrafiltration, taux d'UF,.

volumes d'ultrafiltration,
pression artérielle et veineuse, hématocrite,
volumes sanguins relatifs,
saturation en oxygène,
hémoglobine,
taux d'ultrafiltration,
fréquence cardiaque,
absorbance de toxines urémiques,
pression artérielle systolique,
conductivité du fluide de dialyse (conductivité acide et base, pH), et/ou température du fluide de dialyse, et/ou pour intégrer d'autres techniques d'apprentissage.

# Fig. 1

## Ablaufschema zur Vorhersage des Blutdruckes

# Fig. 2

## UF-Regelung

# Fig. 3

**Möglichkeit einer prospektiven UF-Regelung**

Legende:
- Gewichtsverlustrate
- Systolischer Blutdruck

Y-Achse links: Gewichtsverlustrate (ml/h)
Y-Achse rechts: Systolischer Blutdruck (mmHg)
X-Achse: Zeit (min)

# Fig. 4

**Einfache Darstellung eines künstlichen Neuronalen Netzes**

| Eingabeschicht (Input Layer) | Verdeckte Schicht (Hidden Layer) | Ausgabeschicht (Output Layer) |
| --- | --- | --- |
| 41 | 43 | 45 |

## Fig. 5

### Blutdruck-Vorhersage durch unterschiedliche Netzwerkmodelle

## Fig. 6

### Blutdruck-Vorhersage durch ein Netzwerk mit unterschiedlichen Trainingsalgorithmen

# Fig. 7

**Blutdruck-Vorhersage mit einem NAR-Network**

# Fig. 8

**Blutdruck-Vorhersage mit einem NARX-Network**

# Fig. 9

**Korrelationskoeffizienten zwischen hämodynamischen und von den Maschinensensoren aufgenommenen Parametern mit dem systolischen Blutdruck**

# Fig. 10

**Blutdruck-Vorhersage mit und ohne Zeitverzögerung bei verschiedenen Lernalgorithmen**

### Zeitabhängigkeit

# Fig. 11

**Blutdruck-Vorhersage mit und ohne Zeitverzögerung
bei verschiedenen Lernalgorithmen**

Ensemble-Bildung

Legend:
- ---- Realer Blutdruck
- —— FF-NN Ensemble
- --- TDL-NN Ensemble
- – – SVR Ensemble
- —— SVR-Lag Ensemble
- –·– SVR-Lag & TDL-NN
- ···· SVR & FF-NN

Y-axis: Systolischer Blutdruck (mmHg), X-axis: Ticks

# Fig. 12

**Kontinuierliche Vorhersage einer gesamten
Therapie mit FF-NN**

Legend:
- ---- Realer Blutdruck
- —— Vorhergesagter Blutdruck

Y-axis: Systolischer Blutdruck (mmHg), X-axis: Ticks

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0956872 A2
- EP 1226838 A2
- EP 1844800 B1
- WO 2011080185 A
- WO 2011080190 A
- EP 2061532 B1
- EP 0956872 B1
- US 20070175827 A1